# EUROPEAN PATENT SPECIFICATION

(11) **EP 3 582 746 B1**
(45) Date of publication and mention of the grant of the patent: **10.04.2024**
(21) Application number: 17705133.1
(22) Date of filing: 15.02.2017
(51) Int. Cl.: A61K 8/31, A61K 8/34, A61K 8/37, A61K 8/44, A61K 8/46, A61Q 19/00, A61Q 19/08, A61K 8/891, A61K 8/06

(54) **COSMETIC LOTION**
KOSMETISCHE LOTION
LOTION COSMÉTIQUE

(43) Date of publication of application: 25.12.2019
(73) Proprietor: LVMH Recherche, 45800 Saint Jean de Braye (FR)
(72) Inventor: HATA, Koichi, Tokyo, 102-0092 (JP); OZAWA, Mai, Tokyo, 102-0092 (JP); SAKODA, Takayoshi, Tokyo, 102-0092 (JP)
(74) Representative: Cabinet Beau de Loménie
(86) International application number: PCT/EP2017/053361
(87) International publication number: WO 2018/149491

(56) References cited:
- EP-A1- 3 028 691
- WO-A2-01/08657
- WO-A2-03/024412
- DE-A1-102009 009 004
- JP-A- 2010 173 999

## Description

### Technical Field

The present invention relates to a cosmetic lotion.

### Background Art

Skin care lotions are very fluid products having a high water content and a light texture. They are used to provide the skin with moisture after a cleansing or washing step, which tends to dry the skin up. They can also be used for a gentle cleansing. A fresh watery sensation and a quick penetration sensation are looked for by the consumers who apply a lotion onto their skin. Most of known lotions are aqueous solutions and contain polyols that provide for these properties. Lotion may often lack a nourishing and comfortable feeling.

Cream products have usually more texture than lotions, and essentially produce a substantive skin nourishing effect that is obtained with oily ingredients. Creams soften the skin and give the skin a plumping effect. Most creams contain relatively high oil amounts for that purpose, and may often lack a fresh and quick penetration sensation.

Patent Literature 1 discloses a lotion that contains a polyol(s) and a small amount of an oil ingredient (e.g., one % by mass or less relative to the total mass of the lotion); however, its nourishing effect cannot be said to be sufficient. Therefore, it is desirable to raise the amount of an oil ingredient in a lotion and to improve its nourishing effect and/or confortable feeling sensory properties. However, the higher oil content is used, the lower lotion stability is. A polymer or a thickener is usually added to the lotion to impart viscosity, whereby the oil ingredient can be stabilized in water; however, the fresh watery sensation and the quick penetration sensation that are required for the lotion is impaired in that case. Therefore, the need still exists to provide a stable lotion with a low viscosity comprising a high amount of oil ingredients. A lotion providing a fresh watery sensation, a softening effect, a plumping effect and a nourishing effect at the same time is contemplated as well.

### Citation List

### Patent Literature

Patent Literature 1: Japanese Unexamined Patent Publication No. 2005-255667 Patent Literature 2: WO 03/024412 A2 concerns sprayable oil-in-water low viscosity emulsions.

### Summary of Invention

### Technical Problem

An object of the present invention is to provide a lotion that gives a fresh watery sensation, a quick penetration sensation upon contact with the skin, and a skin-finish sensation that is similar to the sensation one consumer can feel after applying a usual lotion and a usual cream product one after another on skin. By "skin cream finish sensation" is meant a softening, plumping and nourishing effect.
Another object of the present invention is to propose a cosmetic composition showing at least one of the following features: comprising a high amount of water and a high amount of liquid oil ingredients, being stable over time, comprising a low amount of surfactant(s), having a very light texture and having a low viscosity.
The present invention generally relates to an emulsion type lotion comprising: (a) an anionic surfactant; (b) an aliphatic monoalcohol; (c) a polyol; (d) an oil agent; and (e) water, wherein contents of the (c) polyol and the (d) oil agent are, respectively, from 5 to 20% by mass and from 5 to 30% by mass based on a total mass; the (c) polyol comprises from 2 to 6 carbon atoms and from 2 to 4 hydroxyl groups; and an average particle size of emulsion particles composing the emulsion is from 30 to 200 nm.

### Solution to Problem

According to a first embodiment of the present invention, there is provided an emulsion type lotion having an average particle size of oil emulsion particles being from 30 nm to 200 nm, the average particle size being measured using a particle size measuring device according to dynamic light scattering, and said emulsion type lotion comprising at least one anionic surfactant, at least one monoalcohol having a carbon number of from 12 to 24, from 5% to 20% by mass of at least one polyol comprising from 2 to 6 carbon atoms and from 2 to 4 hydroxyl groups, from 5% to 30% by mass of at least one liquid oil agent, and 50% to 90% by mass of water, the percentages by mass being based on the total mass of the lotion, wherein the mass ratio of the liquid oil agent to the anionic surfactant is from 20 to 30.
The emulsion lotion may comprise at least two phases: one oil phase and one water phase. According to one embodiment, an oil phase is dispersed in a water phase and the lotion is an oil-in-water emulsion. The oil phase preferably comprises less than 1% by weight, preferably less than 0.5% by weight, and even preferably is deprived of any solid oil ingredient. It is also preferred that the lotion comprises less than 0.1% by mass, preferably less than 0.01% by mass, of a thickening or gelling agent for water, based on the mass of the lotion. Known thickening agents are for example a carbomer, a carboxymethylcellulose or a xanthan gum. Indeed, the present invention provides a lotion that is stable and very fluid at the same time. The present inventors have surprisingly found that no thickening or gelling agent is required to guarantee lotion stability.

The findings of the present inventors are as follows: In order to form a lotion that can give a skin-finish sensation as if after using cream posterior to application on the skin, it is necessary to raise the content of an oil ingredient in the lotion, while it is demanded that the separation of the oil ingredient is suppressed to stabilize the oil ingredient in water. A polymer or a thickener is added to the lotion to impart a sufficient viscosity, whereby the oil ingredient in water can be stabilized; however, the fresh watery sensation and the quick penetration sensation that are required for the lotion will be impaired. Unexpectedly, the present inventors have found that the lotion employing the above-mentioned configuration solves the above-mentioned problem and makes possible the compatibility of providing the fresh watery sensation and the quick penetration sensation upon contact with the skin and providing the skin-finish nourishing sensation as if after using cream posterior to application on the skin, which means that the lotion softens the skin, provides the skin with plumping and possesses a nourishing effect.

In the lotion of the present invention, the mass ratio of the (d) oil agent to the (a) anionic surfactant is from 20 to 30. The lotion that comprises the anionic surfactant and the oil agent at such a mass ratio is excellent in stability.

In the lotion of the present invention, the (d) oil agent desirably comprises a silicone oil or a hydrocarbon oil. The lotion that comprises one of these oil ingredients is far more stable and can give a good fresh watery sensation and a quicker penetration sensation upon contact with the skin.

The lotion of the present invention desirably has a viscosity of from 1 to 400 mPa.s at 25 °C. The viscosity of the lotion at 25 °C can be measured using a rotational rheometer (e.g., Rheolab QC; manufactured by Anton Paar GmbH; spindle - CC27; and a revolution rate of 200 rpm). If the viscosity is set in such a range, the fresh watery sensation upon contact with the skin will be remarkable.

The lotion of the present invention may further comprise an electrolyte.

### Advantageous Effects of Invention

According to the present invention, there can be provided a lotion that gives a fresh watery sensation and a quick penetration sensation upon contact with the skin as well as gives a skin-finish sensation as if using cream posterior to the application on the skin. Also, the lotion of the present invention is stable after storage without being separated into an oil phase and a water phase. The lotion features three physical properties that were incompatible and not possible to obtain in the same time: stability, low viscosity, high water content and high liquid oil content. The consumer who uses this lotion can feel a fresh sensation, a quick penetration of the product, and a confortable nourishing effect that is similar to the nourishing feeling that may be provided by a textured cream containing a lower water content, and a higher oil content.

### Description of Embodiments

The preferred embodiments of the present invention will be described hereinbelow; however, the present invention shall not be limited to the embodiments below in any way.

The lotion according to the present embodiment preferably comprises (a) an anionic surfactant, as a ionic surfactant. The anionic surfactant can improve stability of oil droplets, improve moisturizing properties, and/or reduce an oily feeling on skin.

Examples of the anionic surfactant include: carboxylic acid-based anionic surfactants such as an aliphatic monocarboxylate, a polyoxyethylene alkyl ether carboxylate, an N-acyl sarcosinate, and an N-acyl glutamate; sulfonic acid-based anionic surfactants such as a dialkylsulfosuccinate, an alkanesulfonate, an α-olefin sulfonate, an alkylbenzene sulfonate, an alkylnaphthalene sulfonate, and a N-methyl-N-acyltaurate; sulfuric acid ester-based anionic surfactants such as an alkyl sulfate and a polyoxyethylene alkyl ether sulfate; and phosphoric acid ester-based anionic surfactants such as an alkylphosphate and a polyoxyethylene alkyl ether phosphate. According to an embodiment, the anionic surfactant is an amino-acid based surfactant selected in the group consisting of glutamates, sarcosinates, alaninates, aspartates and glycinates. N-acyl glutamates having 12 to 22 of carbon atom or salts thereof, such as sodium N-lauroyl glutamate, sodium N-stearoyl-L-glutamate and sodium N-myristoyl-L glutamate. Among these, a N-acylglutamate such as sodium N-stearoyl glutamate, is preferable. One kind of the anionic surfactant may be used alone, or two or more kinds may be used in combination.

The content of the (a) anionic surfactant is preferably from 0.2 to 1.4% by mass, and more preferably, from 0.4 to 1.2% or from 0.8 to 1.0% by mass based on the total mass. Note that as used in the present specification, the term "based on the total mass" means the total mass of the lotion as being the basis.

The lotion according to the present embodiment preferably comprises (b) an aliphatic monoalcohol in addition to the (a) ingredient.
The lotion preferably comprises a surfactant consisting of a mixture of an anionic surfactant and a non ionic surfactant. In this embodiment, the non ionic surfactant can be an aliphatic monoalcohol as mentioned before. The non ionic surfactant can alternatively be a non ionic surfactant having a HLB that is from 8 to 18, preferably from 14 to 16. Combination of an anionic surfactant and a non ionic surfactant is preferable to obtain a stable emulsion lotion providing good sensory feelings during use.

Examples of the aliphatic monoalcohol include aliphatic monoalcohols having a carbon number of from 12 to 24 such as lauryl alcohol, myristyl alcohol, cetyl alcohol (cetanol), stearyl alcohol, behenyl alcohol, oleyl alcohol, and lanoline alcohol. Among these, cetyl alcohol and stearyl alcohol are preferable; and cetyl alcohol is more preferable. One kind of the aliphatic monoalcohol may be used alone, or two or more kinds may be used in combination.

The content of the non ionic surfactant or the content of the (b) aliphatic monoalcohol is preferably from 0.2 to 1.4% by mass, and more preferably, from 0.4 to 1.2% or from 0.8 to 1.0% by mass based on the total mass. Further, in the lotion, the mass ratio of the non ionic surfactant or (b) aliphatic monoalcohol to the (a) anionic surfactant is preferably from 0.1 to 2, and more preferably, from 0.5 to 1.7.

The lotion according to the present embodiment preferably comprises (c) a polyol in addition to the (a) and the (b) ingredients.

The polyol contained in the lotion according to the present embodiment comprises from 2 to 6 carbon atoms and from 2 to 4 hydroxyl groups. Examples of such a polyol include ethylene glycol, 1,3-propanediol, polypropylene glycol, butylene glycol, pentylene glycol, hexylene glycol, dipropylene glycol, glycerin and diglycerin. Among these, butylene glycol, pentylene glycol, glycerin, and a combination of the foregoing are preferable. One kind of the polyol may be used alone, or two or more kinds may be used in combination. A mixture of glycerin, butylene glycol and pentylene glycol illustrates one example of the invention.

The content of the (c) polyol is from 5 to 20% by mass based on the total mass. By letting the content of the polyol be in the range, it is possible to maintain the stability of the lotion without causing gelation, particularly, even at a low temperature. The content of the polyol is preferably from 8 to 18% by mass or from 10 to 20% by mass, more preferably from 10 to 15% by mass, based on the total mass. Further, in the lotion, the mass ratio of the (c) polyol to the (a) anionic surfactant is preferably from 5 to 40, and more preferably, from 10 to 20. If the mass ratio of the (c) ingredient to the (a) ingredient is set in the range, a lotion that is excellent in stability can be formed.

The lotion according to the present embodiment comprises (d) an oil agent in addition to the (a) to (c) ingredients. Note that as used in the present specification, the (d) oil agent does not encompass the (b) aliphatic monoalcohol.

The oil agent can be a liquid oil, a solid oil or a mixture thereof. It is preferred that the lotion of the present invention contains a very low amount of a solid oil agent, because it may impair the sensory properties of the product on skin, and increase viscosity thereof. Examples of the oil agent include a fat, a wax, a hydrocarbon liquid oil, a fatty acid, a silicone liquid oil and mixtures thereof. A hydrocarbon oil, in the sense of the invention, is an oil comprising no silicone atom, preferably either an oil consisting of carbon and hydrogen atoms (alkane oil) or an oil consisting of carbon atom, hydrogen and oxygen atoms. The liquid oil agent preferably has a viscosity from 5 to 120 mPa.s at 25°C and atmospheric pressure, said viscosity being measured according to a method that is known from one skilled in the art.

Some examples of a liquid hydrocarbon oil include volatile oils and non volatile oils. A non volatile oil is an oil having an evaporation rate according to DIN 53249 at 25°C that is lower than that of isododecane at the end of a 5 minute time period. The hydrocarbon liquid oil is preferably a non volatile liquid oil.
According to one embodiment, the hydrocarbon liquid oil is selected in the group consisting of alkane liquid oils (consisting of carbon and hydrogen atoms), ester liquid oils (consisting of carbon, hydrogen and oxygen atoms and having at least one ester group); and mixtures thereof.
Alkane liquid oils include liquid paraffin, light liquid isoparaffin, dodecane, isododecane, tetradecane, isotetradecane, hexadecane, isohexadecane, squalane, vegetable squalane, vaseline, polyisobutylene, polybutene, hydrogenated polyisobutene, and others. Alkane non volatile liquid oils are preferred.

Concrete examples of the fat include Japan wax, olive oil, castor oil, mink oil, macadamia nut oil, camellia oil, rosehip oil, avocado oil, and others.

Concrete examples of the wax include beeswax, lanoline, carnauba wax, candelilla wax, spermaceti, and others.

Concrete examples of the ester liquid oil include jojoba oil, cetyl isooctanate, cetyl ethylhexanoate, isopropyl myristate, isopropyl palmitate, ethylhexyl palmitate, octyldodecyl myristate, glyceryl trioctate (triethylhexanoin or glyceryl 2-ethyl-hexanoate), polyglyceryl diisostearate, digyceryl triisostearate, glyceryl tribehenate, diisostearyl malate, neopentylglycol dioctanate, neopentylglycol dicaprate, caprylic/capric triglyceride, a cholesterol fatty acid ester, and others. Low polar and low viscosity esters are preferred.

Concrete examples of the fatty acid include lauric acid, myristic acid, palmitic acid, stearic acid, behenic acid, oleic acid, and others.

Concrete examples of the liquid silicone oil include polydimethylsiloxane (dimethicone), polymethylphenylsiloxane (diphenyldimethicone), phenyltrimethicone, diphenylsiloxyphenyltrimethicone, an amino-modified silicone, an epoxy-modified silicone, a carboxy-modified silicone, a polyether-modified silicone, an alkyl-modified silicone, and others. The liquid silicone oil is preferably non volatile.

The (d) oil agent preferably comprises a liquid hydrocarbon oil, a liquid silicone oil, or a mixture thereof. If the oil agent comprises either of these components, a good fresh watery sensation and a quicker penetration sensation can be given upon contact with the skin, and a more stable lotion can be provided. One kind of the oil agent may be used alone, or two or more kinds may be used in combination.
The oil agent is preferably a liquid oil having a viscosity from 5 to 120 mPa.s at 25°C and atmospheric pressure that is selected in the group consisting of a dimethicone, a phenyltrimethicone, an alkane, an ester, or mixture thereof. According to several distinct embodiments, the oil agent consists of a liquid silicone oil, a liquid alkane oil, a mixture of a liquid silicone oil and a liquid ester oil, or a mixture of a liquid alkane oil and a liquid ester oil. For example, the liquid oil agent consists of squalane, the liquid oil agent consists of dimethicone; the liquid oil agent consists of phenyltrimethicone, or the liquid oil agent consists of dimethicone and ester liquid oil, such as described before.

The content of the (d) oil agent is preferably from 5 to 30% by mass based on the total mass. Such an oil agent content makes it possible to obtain both a fresh watery sensation upon contact with the skin and a skin cream finish sensation. The content of the oil agent is preferably from 10 to 25% by mass based on the total mass. Further, in the lotion, the mass ratio of the (d) oil agent to the (a) anionic surfactant is preferably from 20 to 30, and more preferably, from 20 to 25. If the mass ratio of the (d) ingredient to the (a) ingredient is set in the ranges, a lotion that is excellent in stability can be formed.

In the lotion according to the present embodiment, the respective mass ratios of the (b) aliphatic monoalcohol and the (d) oil agent to the (a) anionic surfactant are preferably from 0.1 to 2 and from 20 to 30, and more preferably, from 0.5 to 1.7 and from 20 to 25.

The lotion according to the present embodiment comprises (e) water in addition to the (a) to (d) ingredients. As the water, there can be used steam-distilled water that is derived from plants, which includes lavender water, rose water, and orange flower water, apart from distilled water, purified water, hot spring water, and deep water. Water can be from 50 to 90% by mass, for example from 60 to 70% by mass, relative to the mass of the lotion.

A second embodiment of the present invention relates to an oil-in-water emulsion comprising one anionic surfactant, one non-ionic surfactant having a HLB that is from 8 to 18, 10 to 15% by weight of at least one polyol, 15 to 25% by weight of a liquid oil ingredient, and 50 to 90% by weight of water, the percentages being based on the weight of the emulsion, wherein the liquid oil ingredient is selected in the group consisting of apolar liquid oils, low polar liquid oils and mixtures thereof, and wherein the liquid oil ingredient has a viscosity being from 10 to 120 mPa.s at 25°C and atmospheric pressure, the viscosity being measured according to a method that is known from one skilled in the art. In this embodiment, the non ionic surfactant can be a saturated monoalcohol having a carbon number of from 12 to 24, the apolar liquid oil can be a silicone or an alkane, and the low polar liquid oil can be an ester bearing no alcohol group.
A particular aspect of the invention relates to an oil-in-water emulsion comprising from 0.5 to 1.5% by mass of amino-acid based surfactant, from 0.5 to 1.5% by mass of a saturated monoalcohol having a carbon number of from 12 to 24, from 10 to 15% by weight of at least one polyol, preferably a mixture a glycerin, butylene glycol and pentylene glycol, 15 to 25% by weight of a liquid oil ingredient selected in the group consisting of silicone liquid oils, alkane liquid oils, non hydroxylated ester liquid oils and mixtures thereof, and 50 to 90% by weight of water, the percentages being based on the weight of the emulsion. The liquid oil ingredient is preferably non volatile. The liquid oil preferably forms droplets in water, said droplets having an average size being from 70 to 160 nm. The emulsion preferably has a viscosity being from 6 to 60 mPa.s. All the features that have been described in relation to the first embodiment above can apply to this second embodiment.

In the emulsion type lotion according to the present embodiment, the average particle size of emulsion particles composing the emulsion is preferably from 30 to 200 nm. Emulsion particles in the sense of the invention can be oil droplets that are dispersed in water. By letting the average particle size be within the range, it will be possible to provide a stable lotion that can give the fresh watery sensation and the quick penetration sensation upon contact with the skin. The average particle size of the emulsion particles is preferably from 30 to 180 nm, and more preferably, from 50 to 160 nm. The average particle size of the emulsion particles can be measured using a particle size measuring device (e.g., DelsaMax CORE: manufactured by Beckman Coulter, Inc.) according to dynamic light scattering. Further, a high-pressure emulsifying apparatus (e.g., Star Burst: manufactured by Sugino Machine Limited) can be used to process a mixture comprising the (a) to (e) ingredients above (with the (f) electrolyte and other additives as necessary) in plural times at a high pressure (preferably 200 MPa or greater), whereby it is possible to set the average particle of the emulsion particles in the lotion within the above-mentioned range.

The viscosity of the lotion according to the present embodiment can be set in the range of from 1 to 400 mPa.s at 25 °C. By letting the viscosity range be in such a range, it is possible to impart the skin with a marked fresh watery sensation upon contact. The viscosity at 25 °C is more preferably from 5 to 200 mPa.s, and further more preferably, from 10 to 100 mPa.s. The viscosity can be measured using the rotational rheometer (e.g., Rheolab QC; manufactured by Anton Paar GmbH; spindle - CC27; and a revolution rate of 200 rpm). The viscosity of the lotion can advantageously be from 10 to 15 mPa.s.

The lotion according to the present embodiment may further comprise (f) an electrolyte. If the (a) to (e) ingredients described above are combined even when the lotion comprises the electrolyte, a stable lotion can be provided.

Examples of the electrolyte include edetic acid, citric acid, lactic acid, glycolic acid, succinic acid, tartaric acid, malic acid, ascorbic acid, and salts of the foregoing. Other examples of the electrolyte are potassium citrate, disodium EDTA and tetrasodium EDTA, disodium EDTA and disodium phosphate. One kind of the electrolyte may be used alone, or two or more kinds may be used in combination.

The lotion according to the present embodiment can appropriately be compounded with additives in order to impart the lotion with various effects, which include an antioxidant, a brown-discoloration preventer, a cosmetically-active ingredient, a dye, a perfume, and the like, in addition to the above-mentioned ingredients. The lotion can also comprise preservatives such as, but not limited to phenoxyethanol, benzyl alcohol, dichlorobenzyl alcohol, sorbic acid and potassium sorbate.

The lotion according to the present embodiment can be produced by following the steps below, for example.
(1) The anionic surfactant, the polyol, water, and if necessary, the above-mentioned additives that are water-based are mixed and stirred so as to be wholly homogeneous, whereby a water-based mixture is obtained.
(2) The aliphatic monoalcohol, the oil agent, and if necessary, the electrolyte and the above-mentioned additives that are oil-based are mixed and stirred so as to be wholly homogeneous, whereby an oil-based mixture is obtained.
(3) The water-based mixture obtained in (1) above was stirred with a homogenizer, to which is added the oil-based mixture obtained in (2), followed by further stirring.
(4) The mixture obtained in (3) above is processed in plural times at a high pressure (preferably, 200 MPa or greater) using a high-pressure emulsifying apparatus (e.g., Star Burst: manufactured by Sugino Machine Limited).
The invention also describes a process for preparing the emulsion as described above. This process comprises a step of preparing a water phase comprising the anionic surfactant, the polyol and water at a temperature that is between 70 and 80°C, a step of preparing an oil phase comprising the non ionic surfactant and the oil ingredient at a temperature that is between 70 and 80°C, a step of mixing the water phase and the oil phase at a temperature that is between 70 and 80°C and with stirring at a revolution speed higher than 10,000 rpm, a step of allowing the mixture to cool down to 25°C with further stirring at a revolution speed higher than 10,000 rpm, and a step of emulsifying the mixture at a pressure higher than 200 MPa in a high-pressure emulsifying apparatus. A high-pressure emulsifying step is preferred in a view to obtain oil droplets having an average size between 30 and 200 nm that are dispersed in water in a stable way.
In the sense of the invention, "stability" or "stable lotion" can be observed in the following lotion storage conditions: storage at 50 °C and 4 °C for one month, storage at -18 °C for one week; and/or storage with temperature variation cycles (for example a cycle at 40 °C for 12 hours and -10 °C for 12 hours, that is repeated for one month).

### EXAMPLES

Hereinbelow, the lotion will be further described by way of the examples of the present invention; however, the present invention is not limited to the examples below.

### (1) Preparation of Emulsion Type Lotions

The anionic surfactant, the polyols, water, the electrolytes, and phenoxyethanol, which are shown in Tables 1 to 3, were mixed, and stirred so as to be wholly homogeneous at 75 °C, whereby a water-based mixture was obtained. Further, the aliphatic monoalcohol and the oil agent(s), which are shown in Tables 1 to 3, were mixed, and stirred so as to be wholly homogeneous at 75 °C, whereby an oil-based mixture was obtained. The obtained water-based mixture was stirred with the homogenizer (T25 digital ULTRA-TURRAX: manufactured by IKA Werke GmbH & Co.; and a revolution speed of 12,000 rpm) for 5 minutes, to which was added the oil-based mixture obtained, followed by further stirring for 5 minutes. After having been cooled to room temperature, the obtained mixture was processed three times at a pressure of 200 MPa using the high-pressure emulsifying apparatus (Star Burst: manufactured by Sugino Machine Limited) to prepare the respective lotions of the Examples and the Comparative Examples. The compounding ratios (% by mass) of the respective ingredients are as shown in Tables 1 to 3. Note that the lotions of Comparative Examples 1 and 2 are those which have not been processed by the high-pressure emulsifying apparatus in Examples 2 and 3, respectively.

### (2) Measurement of Average Particle Sizes

The average particle size of the emulsion particles in each lotion prepared above was measured using a particle size measuring device (DelsaMax CORE: manufactured by Beckman Coulter, Inc.) according to dynamic light scattering.

### (3) Viscosity Measurement

The viscosity of each lotion prepared above at 25 °C was measured using the rotational rheometer (Rheolab QC; manufactured by Anton Paar GmbH; spindle - CC27; and a revolution rate of 200 rpm).

### (4) Stability Evaluation

### (4-1: Storage at 50 °C and 4 °C for one month)

Each lotion prepared above was accommodated in a transparent container, and after having sealed it with a lid, it was stored at 50 °C or 4 °C for one month. After storage, the absence or presence of the separation between an oil phase and a water phase was observed. That for which no separation between an oil phase and a water phase had been observed both at 50 °C and at 4 °C was evaluated "Y"; and that for which the separation between an oil phase and a water phase had been observed either at 50 °C or at 4 °C was evaluated "N."

### (4-2: Storage at -18 °C for one week)

Each lotion prepared above was accommodated in a transparent container, and after having sealed it with a lid, it was stored at -18 °C for one week. After storage, the absence or presence of the separation between an oil phase and a water phase was observed. That for which no separation between an oil phase and a water phase had been observed was evaluated "Y"; and that for which the separation between an oil phase and a water phase had been observed was evaluated "N."

### (4-3: Cycle test at 40 °C and -10 °C)

Each lotion prepared above was accommodated in a transparent container, and after having sealed it with a lid, it was stored at 40 °C for 12 hours and was then stored at -10 °C for 12 hours, which were repeated alternately to store for the total of one month. That for which no separation between an oil phase and a water phase had been observed was evaluated "Y"; and that for which the separation between an oil phase and a water phase had been observed was evaluated "N."

### (5) Sensory evaluation

As for each lotion prepared above, the fresh watery sensation, quick penetration sensation, and the skin-finish sensation as if after using cream were evaluated in a one-day single use test by 10 in-house cosmetic-specialized evaluation panel (age 25-55 years old) of the organization to which the present inventors belong, according to the standard below.

The fresh watery sensation, the quick penetration sensation and the skin finish sensation as if after using cream were evaluated in terms of: "A" - markedly noted; "B" - noted; "C" - not sufficiently noted; "D" - not noted.

**[Table 1]**

| | Ingredients | Example 1 | Example 2 | Example 3 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|
| (a) Anionic surfactant | Sodium stearoyl glutamate (Trade Name: AMISOFT HS-11 PF) | 0.9 | 0.9 | 0.9 | 0.9 | 0.9 | 0.9 | 0.9 | 0.9 | 0.9 | 0.9 |
| (b) Aliphatic monool | Cetyl alcohol | 0.9 | 0.9 | 0.9 | 0.9 | 0.9 | - | - | 0.9 | 0.9 | 0.9 |
| (c) Polyol | Glycerin | 5 | 5 | 5 | 5 | 5 | 5 | 5 | - | - | - |
| | Butylene glycol | 5 | 5 | 5 | 5 | 5 | 5 | 5 | - | - | - |
| | Pentylene glycol | 2 | 2 | 2 | 2 | 2 | 2 | 2 | - | - | - |
| | Sorbitol (Trade Name: NEOSORB 70/02) | - | - | - | - | - | - | - | - | - | 17.2 |
| (d) Oil agent | Dimethicone (Trade Name: XIAMESTER PMX-200 SILICONE FLUID 100S) | 20 | 20 | - | 20 | - | 20 | - | 20 | - | 20 |
| | Squalane | - | - | 20 | - | 20 | - | 20 | - | 20 | - |
| (e) Water | Water | balance | balance | balance | balance | balance | balance | balance | balance | balance | balance |
| (f) Electrolyte | Disodium EDTA | - | 0.05 | 0.05 | 0.05 | 0.05 | 0.05 | 0.05 | 0.05 | 0.05 | 0.05 |
| | Citric acid | - | 0.05 | 0.05 | 0.05 | 0.05 | 0.05 | 0.05 | 0.05 | 0.05 | 0.05 |
| | Phenoxyethanol | 0.8 | 0.8 | 0.8 | 0.8 | 0.8 | 0.8 | 0.8 | 0.8 | 0.8 | 0.8 |
| Total mass (%) | | 100 | 100 | 100 | 100 | 100 | 100 | 100 | 100 | 100 | 100 |
| Average particle size (nm) | | 97 | 107 | 91 | 246 | 763 | 107 | 132 | 133 | 151 | 153 |
| Viscosity (mPas) | | 10.63 | 14.01 | 13.55 | 17.11 | 25.09 | 12.77 | 14.03 | 8.88 | 9.96 | 11.13 |
| Stability evaluation | 50 °C x one month, 4 °C x one month | Y | Y | Y | N | N | N | N | Y | Y | N |
| | -18 °C x one week | Y | Y | Y | N | N | N | N | N | N | N |
| | Cycle at 40 °C and -10 °C | Y | Y | Y | N | N | N | Y | N | N | N |
| Fresh watery sensation | | A | A | A | D | D | B | B | B | B | A |
| Quick penetration sensation | | A | A | A | D | D | D | D | B | B | D |
| Skin-finish sensation as if after using cream | | A | A | A | B | B | B | B | D | D | C |

| | | | | | | | | | | | |
|---|---|---|---|---|---|---|---|---|---|---|---|
| No high pressure emulsifying step has been performed in the process for preparing comparative Example 1 and comparative Example 2. | | | | | | | | | | | |

**[Table 2]**

| | Ingredients | Example 4 | Example 5 | Example 6 | Example 7 | Example 8 | Example 9 |
|---|---|---|---|---|---|---|---|
| (a) Anionic surfactant | Sodium stearoyl glutamate (Trade Name: AMISOFT HS-11 PF) | 0.225 | 1.35 | 0.9 | 0.9 | 0.9 | 0.9 |
| (b) Aliphatic monool | Cetyl alcohol | 0.225 | 1.35 | 0.9 | 0.9 | 0.09 | 1.8 |
| (c) Polyol | Glycerin | 5 | 5 | 5 | 5 | 5 | 5 |
| | Butylene glycol | 5 | 5 | 5 | 5 | 5 | 5 |
| | Pentylene glycol | 2 | 2 | 2 | 2 | 2 | 2 |
| (d) Oil agent | Dimethicone (Trade Name: XIAMESTER PMX-200 SILICONE FLUID 1005) | 5 | 30 | 18 | 27 | 20 | 20 |
| (e) Water | Water | balance | balance | balance | balance | balance | balance |
| (f) Electrolyte | Disodium EDTA | 0.05 | 0.05 | 0.05 | 0.05 | 0.05 | 0.05 |
| | Citric acid | 0.05 | 0.05 | 0.05 | 0.05 | 0.05 | 0.05 |
| | Phenoxyethanol | 0.8 | 0.8 | 0.8 | 0.8 | 0.8 | 0.8 |
| Total mass (%) | | 100 | 100 | 100 | 100 | 100 | 100 |
| Average particle size (nm) | | 140 | 79 | 110 | 150 | 136 | 108 |
| Viscosity (mPas) | | 6.59 | 56.08 | 13.93 | 13.14 | 12.36 | 14.71 |
| Stability evaluation | 50 °C x one month, 4 °C x one month | Y | Y | Y | Y | Y | Y |
| | Cycle at 40 °C and -10 °C | Y | Y | Y | Y | Y | Y |
| Fresh watery sensation | | A | B | B | B | A | B |
| Quick penetration sensation | | A | B | B | B | B | A |
| Skin-finish sensation as if after using cream | | B | A | A | A | A | A |

**[Table 3]**

| | Ingredients | Example 10 | Example 11 |
|---|---|---|---|
| (a) Anionic surfactant | Sodium stearoyl glutamate (Trade Name: AMISOFT HS-11 PF) | 0.9 | 0.9 |
| (b) Aliphatic monool | Cetyl alcohol | 0.9 | 0.9 |
| (c) Polyol | Glycerin | 5 | 5 |
| | Butylene glycol | 5 | 5 |
| | Pentylene glycol | 2 | 2 |
| (d) Oil agent | Dimethicone (Trade Name: XIAMESTER PMX-200 SILICONE FLUID 1005) | - | 10 |
| | Phenyltrimethicone | 20 | - |
| | Triethylhexanoin | - | 10 |
| (e) Water | Water | balance | balance |
| (f) Electrolyte | Disodium EDTA | 0.05 | 0.05 |
| | Citric acid | 0.05 | 0.05 |
| | Phenoxyethanol | 0.8 | 0.8 |
| Total mass (%) | | 100 | 100 |
| Average particle size (nm) | | 104 | 92 |
| Viscosity (mPas) | | 13.27 | 12.36 |
| Stability evaluation | 50 °C x one month, 4 °C x one month | Y | Y |
| | Cycle at 40 °C and -10 °C | Y | Y |
| Fresh watery sensation | | A | A |
| Quick penetration sensation | | A | A |
| Skin-finish sensation as if after using cream | | A | A |

## Claims

1. An emulsion type lotion having an average particle size of oil emulsion particles being from 30 nm to 200 nm, the average particle size being measured using a particle size measuring device according to dynamic light scattering, and said emulsion type lotion comprising at least one anionic surfactant, at least one monoalcohol having a carbon number of from 12 to 24, from 5% to 20% by mass of at least one polyol comprising from 2 to 6 carbon atoms and from 2 to 4 hydroxyl groups, from 5% to 30% by mass of at least one liquid oil agent, and 50% to 90% by mass of water, the percentages by mass being based on the total mass of the lotion, wherein the mass ratio of the liquid oil agent to the anionic surfactant is from 20 to 30.

2. The lotion according to claim 1, wherein the oil agent comprises a silicone oil or a hydrocarbon oil.

3. The lotion according to claim 1 or 2, having a viscosity of from 1 mPa.s to 400 mPas at 25 °C.

4. The lotion according to any one of claims 1 to 3, further comprising an electrolyte.

5. The lotion according to any one of claims 1 to 4, wherein the average particle size of oil emulsion particles is from 30 to 180 nm.

6. The lotion according to claim 2, wherein the oil agent is selected in the group consisting of silicone liquid oils, alkane liquid oils, non-hydroxylated ester liquid oils and mixtures thereof.

7. The lotion according to claim 6, wherein the liquid oil agent is selected in the group consisting of a dimethicone, squalane, a phenyltrimethicone, triethylhexanoin and mixtures thereof.

8. The lotion according to claim 1, having a viscosity of from 1 mPa.s to 400 mPa.s at 25°C.

9. The lotion according to any one of claims 1 to 4, wherein the monoalcohol is cetyl alcohol.

10. The lotion according to any one of claims 1 to 5, wherein the polyol is a mixture of glycerin, pentylene glycol and butylene glycol.

11. The lotion according to claim 1, wherein the emulsion is an oil-in-water emulsion.

## Patentansprüche

1. Lotion vom Emulsionstyp mit einer durchschnittlichen Partikelgröße von Ölemulsionspartikeln von 30 nm bis 200 nm, wobei die durchschnittliche Partikelgröße unter Verwendung einer Partikelgrößenmessvorrichtung gemäß dynamischer Lichtstreuung gemessen wird, und wobei die Lotion vom Emulsionstyp zumindest ein anionisches Tensid, zumindest einen Monoalkohol mit einer Kohlenstoffzahl von 12 bis 24, 5 Masse-% bis 20 Masse-% zumindest eines Polyols mit 2 bis 6 Kohlenstoffatomen und 2 bis 4 Hydroxylgruppen, 5 Masse-% bis 30 Masse-% zumindest eines Flüssigölmittels und 50 Masse-% bis 90 Masse-% Wasser umfasst, wobei sich die Masseprozentsätze auf die Gesamtmasse der Lotion beziehen, wobei das Masseverhältnis des Flüssigölmittels zu dem anionischen Tensid 20 bis 30 beträgt.

2. Lotion nach Anspruch 1, wobei das Ölmittel ein Silikonöl oder ein Kohlenwasserstofföl umfasst.

3. Lotion nach Anspruch 1 oder 2 mit einer Viskosität von 1 mPa.s bis 400 mPas bei 25 °C.

4. Lotion nach einem der Ansprüche 1 bis 3, die ferner einen Elektrolyten umfasst.

5. Lotion nach einem der Ansprüche 1 bis 4, wobei die durchschnittliche Partikelgröße von Ölemulsionspartikeln 30 bis 180 nm beträgt.

6. Lotion nach Anspruch 2, wobei das Ölmittel aus der Gruppe ausgewählt ist, die aus Silikonflüssigölen, Alkanflüssigölen, nichthydroxylierten Esterflüssigölen und Gemischen davon besteht.

7. Lotion nach Anspruch 6, wobei das Flüssigölmittel aus der Gruppe ausgewählt ist, die aus einem Dimethicon, Squalan, einem Phenyltrimethicon, Triethylhexanoin und Gemischen davon besteht.

8. Lotion nach Anspruch 1 mit einer Viskosität von 1 mPa.s bis 400 mPa.s bei 25 °C.

9. Lotion nach einem der Ansprüche 1 bis 4, wobei der Monoalkohol Cetylalkohol ist.

10. Lotion nach einem der Ansprüche 1 bis 5, wobei das Polyol ein Gemisch von Glycerin, Pentylenglycol und Butylenglycol ist.

11. Lotion nach Anspruch 1, wobei die Emulsion eine Öl-in-Wasser-Emulsion ist.

## Revendications

1. Lotion de type émulsion ayant une taille de particule moyenne des particules d'émulsion huileuse de 30 nm à 200 nm, la taille de particule moyenne étant mesurée au moyen d'un dispositif de mesure de taille de particule conformément à une diffusion dynamique de la lumière, ladite lotion de type émulsion comprenant au moins un tensioactif anionique, au moins un alcool monovalent ayant de 12 à 24 carbones, 5 % à 20 % en masse d'au moins un polyol comprenant 2 à 6 atomes de carbone et 2 à 4 groupes hydroxyle, 5 % à 30 % en masse d'au moins un agent huileux liquide, et 50 % à 90 % en masse d'eau, les pourcentages en masse étant basés sur la masse totale de la lotion, dans laquelle le rapport en masse de l'agent huileux liquide au tensioactif anionique est de 20 à 30.

2. Lotion selon la revendication 1, dans laquelle l'agent huileux comprend une huile de silicone ou une huile d'hydrocarbure.

3. Lotion selon la revendication 1 ou 2, ayant une viscosité de 1 mPa.s à 400 mPa.s à 25°C.

4. Lotion selon l'une quelconque des revendications 1 à 3, comprenant en outre un électrolyte.

5. Lotion selon l'une quelconque des revendications 1 à 4, dans laquelle la taille de particule moyenne des particules en émulsion huileuse est de 30 à 180 nm.

6. Lotion selon la revendication 2, dans laquelle l'agent huileux est choisi dans le groupe constitué par les huiles de silicone liquides, les huiles d'alcane liquides, les huiles d'ester non hydroxylé liquides et leurs mélanges.

7. Lotion selon la revendication 6, dans laquelle l'agent huileux liquide est choisi dans le groupe constitué par une diméthicone, le squalane, une phényltriméthicone, la triéthylhexanoïne et leurs mélanges.

8. Lotion selon la revendication 1, ayant une viscosité de 1 mPa.s à 400 mPa.s à 25°C.

9. Lotion selon l'une quelconque des revendications 1 à 4, dans laquelle l'alcool monovalent est l'alcool cétylique.

10. Lotion selon l'une quelconque des revendications 1 à 5, dans laquelle le polyol est un mélange de glycérol, de pentylèneglycol et de butylèneglycol.

11. Lotion selon la revendication 1, dans laquelle l'émulsion est une émulsion huile dans l'eau.
